Europäisches Patentamt

European Patent Office

Office européen des brevets

(11) Veröffentlichungsnummer: **0 343 479**

**A1**

(12) **EUROPÄISCHE PATENTANMELDUNG**

(21) Anmeldenummer: 89108706.6

(22) Anmeldetag: 16.05.89

(51) Int. Cl.⁴: **A61K 39/395** , //(A61K39/395, 37:02)

Patentanspruch für folgenden Vertragsstaat: ES

(30) Priorität: 27.05.88 DE 3818055

(43) Veröffentlichungstag der Anmeldung:
29.11.89 Patentblatt 89/48

(84) Benannte Vertragsstaaten:
AT BE CH DE ES FR GB GR IT LI LU NL SE

(71) Anmelder: **BIOTEST PHARMA GMBH**
**Landsteiner Strasse 5**
**D-6072 Dreieich(DE)**

(72) Erfinder: **Schwulera, Udo, Dr. Dipl.-Biol.**
**Krebsbachweg 23**
**D-6450 Hanau 1(DE)**
Erfinder: **Dichtelmüller, Herbert, Dr.**
**Molekular-Biol.**
**Rossertstrasse 14**
**D-6231 Sulzbach/Ts.(DE)**
Erfinder: **Stephan, Wolfgang, Dr. Dipl.-Chem.**
**Philipp-Holzmann-Strasse 84**
**D-6072 Dreieich(DE)**
Erfinder: **Thrun, Alexander, Dr. Dipl.-Biol.**
**Konrad-Duden-weg 39**
**D-6000 Frankfurt/M. 56(DE)**
Erfinder: **Lissner, Reinhard, Dr. Dipl.-Chem.**
**Gönz 18**
**D-8761 Weilbach(DE)**

(74) Vertreter: **Beil, Hans Chr., Dr. et al**
**Beil, Wolff und Beil, Rechtsanwälte**
**Adelonstrasse 58 Postfach 80 01 40**
**D-6230 Frankfurt am Main 80(DE)**

(54) Kombination aus humanen Immunglobulinen und Interleukin-2 zur Bekämpfung von Infektionskrankheiten.

(57) Eine verbesserte immunbiologische Wirksamkeit wird erzielt durch die Verwendung einer Kombination von humanem Immunglobulin und humanem Interleukin-2 als immunbiologisch wirksames Mittel bei der Bekämpfung von Infektionskrankheiten.

EP 0 343 479 A1

## Kombination aus humanen Immunoglobulinen und Interleukin-2 zur Bekämpfung von Infektionskrankheiten

Der Erfindung betrifft die Verwendung einer Kombination von humanem Immunglobulin und Interleukin-2 als immunbiologisch wirksames Mittel bei der Bekämpfung von Infektionskrankheiten.

Interleukin-2 wurde zuerst 1976 von morgen et al.(1) in den Kulturüberständen von lektinaktiviertem humanen peripheren mononukleären Zellen als T-Zellwachstumsfaktor (TCGF) entdeckt, der die Kultur von aktivierten T-Lymphozyten erstemals ermöglichte. Die Bezeichnung Interleukin-2 für T-Zellwachstumsfaktor prägte sich auf dem Zweiten Internationalen Workshop in Ermatingen (2).

Humanes Interleukin-2 aus peripheren Blutlymphozyten gewonnen, ist ein stark hydrophobes Glykoprotein mit einem Molekulargewicht zwischen 14.500 bis 17.000, abhängig vom Glykosylierungsgrad. Das Interleukin-2-Gen ist auf Chromosom 6 lokalisiert. Taniguchi und Mitarbeiter (3) konnten zeigen, dass Interleukin-2 aus einer einzigen Polypeptidkette mit 133 Aminosäuren besteht und drei Cystein-Reste besitzt, wobei zwei eine intramolekulare Disulfid-Brücke bilden, die essentiell für die biologische Aktivität ist.

Um seine biologische Wirkung entfalten zu können, muss IL-2 sich an seinen Rezeptor anlagern. Nach Internalisierung des Peptidhormons findet über einen noch unbekannten Mechanismus die Transduktion des Signals statt, die letztendlich die Zellteilung initiiert.

Die biologischen Wirkungen von Interleukin-2 sind vielfältig. Neben der schon erwähnten Eigenschaft als T-Zellwachstumsfaktor gibt es auch prolifierationsunabhängige Effekte:
- Steigerung der cytotoxischen Aktivität von natürlichen Killerzellen (NK-Zellen)
- Induktion der cytotoxischen Aktivität von Lymphokin-aktivierten Killerzellen (LAK-Zellen)
- Differenzierung von Thymozyten zu cytotoxischen T-Lymphozyten
- Aktivierung der Synthese von Interferon-Gamma (siehe auch Fink et al. (4)).

Diese in-vitro und in-vivo nachgewiesenen biologischen Aktivitäten zeigen, dass Interleukin-2 eine zentral Rolle bei der T-zellabhängigen Immunität spielt und in erster Linie die zelluläre Immunantwort verstärkt. Es ist deshalb nicht zu erwarten, dass das humorale Immunsystem mit Interleukin-2 zusammen wirkt.

Immunoglobulin-Präparate grundsätzlich dort zur Anwendung, wo das humorale Immunsystem eines Patienten nicht über genügend Antikörper verfügt, um das damit verbundene Infektionsrisiko zu reduzieren. Deshalb werden Immunoglobuline bei folgenden Indikationen eingesetzt:
- Zur Prophylaxe bei infektionsanfälligen Patienten, die über keinen oder nur einen geringen Antikörpertiter gegenüber dem Krankheitserreger verfügen bzw.
- zur Substitution bei Patienten, die aufgrund eines erworbenen oder angeborenen Defektes des humoralen Immunsystems nicht in der Lage sind, Antikörper zu bilden oder aufgrund einer z.B. äusseren Einwirkung einen Mangel aufweisen;
- zur Therapie bei Patienten nach einer Operation, nach einem Unfall, nach immunsuppressiver Therapie, bei postoperativer Komplikation, z.B. Sepsis, um einem erhöhten Antikörperverbrauch Rechnung zu tragen.

Man ist laufend bestrebt, Mittel zu finden, deren immunbiologische Wirksamkeit bei der Bekämpfung von Infektionskrankheiten verbessert ist.

So ist die Kombination von Immunoglobulin mit Antibiotika und/oder anderen pharmakologish wirksamen Stoffen beschrieben worden.

Die Immunglobulin-bedingte Agglutination der bakteriellen Antigene führt zu einer Bindung, Erkennung, Präsentation und Beseitigung der Fremdproteine durch Antigen-phagozytierende und -pinozytierende Immunzellen.

Der Erfindung liegt die Aufgabe zugrunde, Mittel mit verbesserter immunbioligischer Wirksamkeit zu finden.

Diese Aufgabe wird durch den Gegenstand gemäss Anspruch 1 gelöst.

Überraschend wurde gefunden, dass eine Kombination von Immunglobulin mit Interleukin-2 eine Steigerung des Wirkung im synergistischen Sinne bewirkt, obwohl Interleukin-2 allein appliziert in verschiedenen Konzentrationen keinen Einfluss auf das humorale Immunsystem hat. Dies zeigt, dass überraschenderweise Interleukin-2 als zellulärer Immunmediator auch auf die B-zellvermittelte humorale Abwehr einen positiven Einfluss hat.

Die erfindungsgemässen Mittel eignen sich für die Bekämpfung sowohl von mikrobiellen als auch viralen Infektionen.

Sie lassen sich sowohl für die Therapie als auch für die Prophylaxe verwenden.

Als Interleukin-2 lassen sich humanes n IL-2 un r IL-2 verwenden.

Zur Erzielung eines Schutzeffektes ist eine Mindestdosis von Interleukin-2 erforderlich. Bei Tierversuchen an der Maus stellte man fest, dass eine Dosis unterhalb von etwa 100 Units pro Tier keine Wirkung zeigt. Bei Zugrundelegung eines Körpergewichts der Maus von 20-30 g kann man daraus eine Mindestmenge von etwa $3\text{-}5\text{x}10^3$ U/kg ableiten. Nach oben hin findet keine Begrenzung statt, jedoch stellte man fest, dass oberhalb einer Menge von $1,5\text{x}10^4$ Units pro Maus keine wesentliche Steigerung mehr stattfindet. Der bevorzugte Mengenbereich des Interleukins liegt somit etwa zwischen $10^3$ U/kg bis $10^7$ U/kg. Die im vorliegenden verwendete Einheit "Units" errechnet sich aus dem Vergleich zur BRMP Referenz (5).

Als Immunglobuline kommen alle humanen Immunglobuline in Betracht. Bevorzugt wird jedoch IgG sowie ein Gemisch aus IgG, IgA und IgM.

Die Menge an Immunglobulin unterliegt keiner besonderen Begrenzen. Aus experimentellen Gründen wurden bei der Maus 0,015 mg/Tier gewählt (0,3 ml einer 5%igen Lösung). Geeignete Dosen liegen bei etwa 0,25 mg/kg bis 15 mg/kg.

Der vorstehend angegebene Bereich an Interleukin-2 lässt sich beliebig mit dem geeigneten Bereich von Immunglobulinen kombinieren. Als besonders geeignet erwies sich eine Kombination von $10^5$ U/kg IL-2 und 0,75 mg/kg Immunglobulin.

Im erfindungsgemässen Mittel können das Interleukin-2 und das Immunglobulin zusammen oder in getrennten Phasen vorliegen. Beide Phasen können in beliebiger Reihenfolge nacheinander verabreicht werden, sie können jedoch auch als Kombination zur Anwendung kommen. Die Präparate können in flüssiger Form oder als Lyophilisat vorliegen, das vorher zu lösen ist oder an einen Träger gebunden bzw. in Kapseln eingeschlossen ist.

Nachstehende Beispiele dienen der weiteren Erläuterung der Erfindung.

### Beispiel 1

Die Wirksamkeit von Immunglobulin und Interleukin-2 wurde in Mäuse-Infektionsschutzversuchen geprüft. Zum Nachweis des syngergistischen Effekts wurde eine suboptimale Immunglobulin-Konzentration gewählt. NMRI-SPF Mäuse wurden mit Salmonella typhimurium mit $3 \times 10^3$ Keinem/Tier intraperitoneal infiziert. Der Versuch umfasste insgesamt 4 Gruppen mit je 30 Tieren.

Als Immunglobulin wurde eine 5%ige Lösung eines Gemischs aus humanem IgG, IgM und IgA verwendet.

Als Interleukin-2 wurde humanes n IL-2 verwendet.

- Gruppe A: Immunglobulin behandelt (pro Tier 0,3 ml einer 5%igen Lösen) und Interleukin-2 (pro Tier 15.000 Units)
- Gruppe B: nur Immunglobulin behandelt (pro Tier 0,3 ml einer 5%igen Lösung)
- Gruppe C: nur Interleukin-2 behandelt (pro Tier 15.000 Units)
- Gruppe D: unbehandelte Kontrolle.

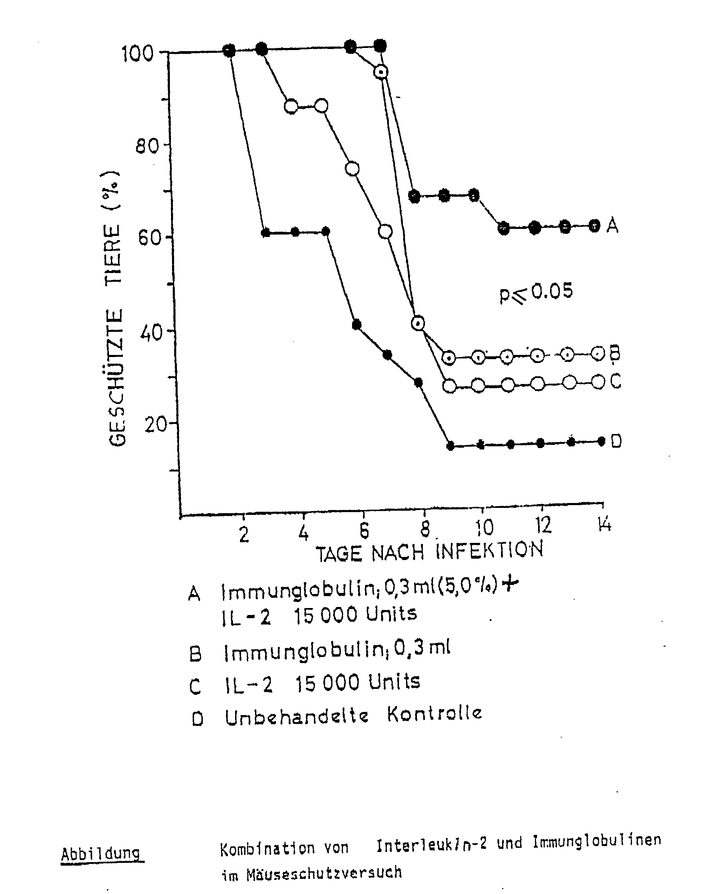

Die Tiere der Gruppen A - C wurden 2 Stunden nach der Infektion intraperitoneal mit den oben angeführten Präparaten behandelt. Als Kirterium der Wirksamkiet diente die Anzahl der durch das jeweilige Präparat 8 tage nach der Infektion noch überlebenden, geschützten Tiere. Das Ergebnis ist in Abbildung 1 dargestellt.

Der beobachtete Unterschied zwischen der Gruppe B/C und auf der anderen Seite Gruppe A ist im $\chi^2$-Test (Chiquadrat-Test (vgl. E.Weber, "Grundriss der biologischen Statistik", 7. Aufl., Gustav Fischer Verlag, S.500-515)) signifikant. Obwohl Interleukin-2 allein nur einen geringen Schutzeffekt zeigt, verstärkt überraschenderweise die Kombination mit Immunglobulinen die Wirksamkeit beträchtlich. Durch die Gabe von Immunglobulin alleine konnten 32% der Tiere, durch alleinige Interleukin-2-Gabe 27% der Tiere, durch Kombination beider Präparate jedoch 63% der Tiere vor Infektion geschützt werden.

### Beispiel 2

Versuch wie 1, jedoch mit steigender Dosierung von Interleukin-2. Der gefundene Schutzeffekt durch Interleukin-2 ist dosisabhängig; unterhalb von 100 Units Interleukin-2 pro Tier ist jedoch keine Wirkung zu beobachten und oberhalb von 15.000 Units pro Tier sieht man keine weitere Steigerung (siehe Tabelle 1).

Tabelle 1

| Kombination von n Interleukin-2 und Immunglobulin im Salmonella typhimurium Infektionsmodell: Dosisabhängigkeit der (synergistischen) Interleukin-2-Wirkung | |
|---|---|
| Interleukin-2 Gesamtdosis in Units pro Tier | Schutzeffekt der Kombination mit Immunglobulinen in Prozent der Maximalwirkung |
| 100 | 5 - 10 % |
| 1.000 | 20 - 30 % |
| 10.000 | 90 - 100 % |
| 50.000 | 100 % |
| 100.000 | 100 % |

Jede Gruppe umfaßt 15 NMRI-Mäuse, die mit je 4,5 x $10^3$ Keimen/Tier i.p. infiziert und gleichzeitig mit Immunglobulin (0,3 ml einer 5 % w/v Lösung) mit der oben angegebenen IL-2 Gesamtdosis/Tier i.p. behandelt wurden.

Beispiel 3

Versuch wie 1, die Interleukin-2 Gabe ist jedoch zeitlich versetzt vor bzw. nach der Infektion. Bei Interleukin-2 Gaben bis zu 5 Stunden vor der Infektion ergab sich eine signifikante Schutzwirkung ($p \leq 0,05$) (siehe Tabelle 2). Bei Interleukin-2 Gaben 2-6h nach der Infektion war ebenfalls eine signifikante Schutzwirkung festzustellen.

Tabelle 2

| Kombination von n Interleukin-2 und Immunglobulin im Salmonella typhimurium-Infektionsmodell: IL-2-Gabe zu verschiedenen Zeitpunkten der Infektion | | | | |
|---|---|---|---|---|
| Therapiegruppe | Überleben (%) nach n Tagen | | | |
| | Tage: 4 (%) | 5 (%) | 7 (%) | 13 (%) |
| IL-2, 5h vor Infektion | 100 | 93 | 60 | 40 |
| IL-2, 3h vor Infektion | 100 | 73 | 53 | 20 |
| IL-2, sofort nach Infektion | 100 | 87 | 47 | 27 |
| Kontrolle, unbehandelt | 90 | 53 | 13 | 0 |

Jede Gruppe umfasst 15 NMRI-Mäuse, die mit je 4,5 x $10^3$ Keimen/Tier i.p.infiziert und davor/gleichzeitig/danach mit Immunglobulin (0,3 ml einer 5% w/v-Lösung) 1,5 x $10^4$ U n IL-2/Tier i.p. zu den angegebenen Zeitpunkten behandelt wurden.

Literatur

1. Morgan, D.A. Ruscetti, F.W., Gallo, R.G. Selective in vitro growth of T-lymphozytes from normal human bone marrow. Science 193 (1976), S.1007

2. Aarden, L.A. Revised nomen dature for antigen-nonspecific T-cell proliferation and helper factors. J. Immunol. 123 (1979), S. 2928

3. Taniguchi, T., Mattni, H., Fujita, T., Takaoka, C., Kashima, N., Yoshimoto, R., Hamuro, J. Structure and expression of a cloned cDNA for human Interleukin-2. Nature 32 (1983),S. 305

4. Fink,R., Dancygier,H. Interleukin-2. DMW 112 (1987), S.188-193

5. Dumonde, D.C., Papermaster, B.W. Availability of a reference reagent of human Interleukin-2. Lymphokin Research $\underline{3}$ (1984), S.227.

## Ansprüche

1. Verwendung einer Kombination von humanen Immunglobulin und humanem Interleukin-2 in Form von n IL-2 und/oder r IL-2 als immunbiologisch wirksames Mittel bei der Bekämpfung von Infektionskrankheiten.

2. Verwendung nach Anspruch 1, dadurch gekennzeichnet, dass das Mittel in einer Verpackungseinheit konfektioniert ist und aus zwei räumlich getrennten Zusammensetzungen besteht, von denen die eine Phase das humane Immunglobulin und die andere Phase das humane Interleukin-2 in Form von n IL-2 und/oder r IL-2 enthält und dass die beiden Phasen gleichzeitig oder beliebig aufeinanderfolgend angewendet werden.

3. Verwendung nach Anspruch 1 oder 2, dadurch gekennzeichnet, dass das Immunglobulin IgG ist.

4. Verwendung nach Anspruch 1 oder 2, dadurch gekennzeichnet, dass sich das Immunglobulin aus IgG, IgA und/oder IgM zusammensetzt.

5. Verwendung nach einem der vorstehenden Ansprüche, dadurch gekennzeichnet, dass das Mischungsverhältnis Ig/IL-2 0,25 mg - 15 mg Ig/$10^2$U-$10^7$ IL-2 beträgt.

6. Verwendung nach Anspruch 5, dadurch gekennzeichnet, dass das Mischungsverhältnis Ig/IL-2 0,75 mg Ig/$10^6$ U IL-2 beträgt.

Patentansprüche für folgenden Vertragsstaat: ES

1. Verfahren zur Herstellung eines immunbiologisch wirksamen Mittels zur Bekämpfung von Infektionskrankheiten, dadurch gekennzeichnet, dass man humanes Immunglobulin und humanes Interleukin-2 in Form von n IL-2 und/oder r IL-2 kombiniert.

2. Verfahren gemäss Anspruch 1, dadurch gekennzeichnet, dass man das Mittel in einer Verpackungseinheit so konfektioniert, dass beide Komponenten getrennt vorliegen, wobei die eine Phase das humane Immunglobulin und die andere Phase das humane Interleukin-2 in Form von n IL-2 und/oder r IL-2 enthält und beide Phasen gleichzeitig oder beliebig aufeinanderfolgend angewendet werden können.

3. Verfahren nach Anspruch 1 oder 2, dadurch gekennzeichnet, dass man das Interleukin-2 in Form von n IL-2 oder r IL-2 mit dem Immunglobulin IgG kombiniert.

4. Verfahren nach Anspruch 1 oder 2, dadurch gekennzeichnet, dass man das Interleukin-2 mit Immunglobulin, bestehend aus IgG, IgA und/oder IgM kombiniert.

5. Verfahren nach einem der vorstehenden Ansprüche 1-4, dadurch gekennzeichnet, dass man das Interleukin-2 und das Immunglobulin im Verhältnis Ig/IL-2 von 0,25 mg bis 15 mg Ig/$10^2$U-$10^7$U IL-2 kombiniert.

6. Verfahren nach Anspruch 5, dadurch gekennzeichnet, dass man Interleukin-2 und das Immunglobulin im Verhältnis Ig/IL-2 von 0,75 mg Ig/$10^6$ U IL-2 kombiniert.

7. Verwendung einer gemäss den Ansprüchen 1-6 hergestellten Kombination von humanem Immunglobulin und humanem Interleukin-2 in Form von n IL-2 und/oder r IL-2 als immunbiologisch wirksames Mittel bei der Bekämpfung von Infektionskrankheiten.

8. Verwendung nach Anspurch 7, dadurch gekennzeichnet, dass das Mittel in einer Verpackungseinheit konfektioniert ist und aus zwei räumlich getrennten Zusammensetzungen besteht, von denen die eine Phase das humane Immunglobulin und die andere Phase das humane Interleukin-2 in Form von n IL-2 und/oder r IL-2 enthält und dass die beiden Phasen gleichzeitig oder beliebig aufeinanderfolgend angewendet werden.

9. Verwendung nach Anspruch 7 oder 8, dadurch gekennzeichnet, dass das Immunglobulin IgG ist.

10. Verwendung nach Anspruch 7 oder 8, dadurch gekennzeichnet, dass sich das Immunglobulin aus IgG, IgA und/oder IgM zusammensetzt.

11. Verwendung nach einem der vorstehenden Ansprüche, dadurch gekennzeichnet, dass das Mischungsverhältnis Ig/IL-2 0,25 mg - 15 mg Ig/$10^2$U-$10^7$ IL-2 beträgt.

12. Verwendung nach Anspruch 11, dadurch gekennzeichnet, dass das Mischungsverhältnis Ig/IL-2 0,75 mg Ig/$10^6$ U IL-2 beträgt.

A  Immunglobulin; 0,3 ml (5,0 %) ✛
   IL-2   15 000 Units

B  Immunglobulin; 0,3 ml

C  IL-2   15 000 Units

D  Unbehandelte Kontrolle

Abbildung        Kombination von    Interleukin-2 und Immunglobulinen
                 im Mäuseschutzversuch

Neu eingereicht / Newly filed
Nouvellement déposé

Europäisches
Patentamt

EUROPÄISCHER RECHERCHENBERICHT

Nummer der Anmeldung

EP 89 10 8706

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 168 830 (BIOTEST PHARMA GmbH)<br>* Seite 1, Zeile 1 - Seite 2, Zeile 19 *<br><br>----- | 1,4 | A 61 K 39/395//<br>(A 61 K 39/395<br>A 61 K 37:02 ) |
| | | | **RECHERCHIERTE SACHGEBIETE (Int. Cl.4)**<br><br>A 61 K |

Der vorliegende Recherchenbericht wurde für alle Patentansprüche erstellt

| Recherchenort | Abschlußdatum der Recherche | Prüfer |
|---|---|---|
| DEN HAAG | 04-09-1989 | BERTOCCHI C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes Dokument

EPO FORM 1503 03.82 (P0403)